# EUROPEAN PATENT APPLICATION

(11) **EP 4 659 650 A1**
(43) Date of publication of application: **10.12.2025**
(21) Application number: 25180810.1
(22) Date of filing: 04.06.2025
(51) Int. Cl.: A61B 1/00, A61B 1/12, A61B 90/70

(54) **AN APPARATUS FOR CLEANSING A SCOPE CONNECTABLE TO THE APPARATUS THROUGH AN ADAPTER THAT CHARACTERIZES SAID SCOPE**

(30) Priority: 06.06.2024 NL 2037877
(71) Applicant: Van Vliet Medical Supply B.V., 1362 JD Almere (NL)
(72) Inventor: HESEL, Ernst Hendrik Anton, Almere (NL); KOPERDRAAT, Bonita, Almere (NL)
(74) Representative: van Breda, Jacobus

(57) **Abstract**

An apparatus (1) for cleansing scopes, such as endoscopes, the apparatus (1) being equipped with a source (3) for supplying cleansing fluid, and an outlet (5) to which said source (3) for cleansing fluid is connected, wherein a fluid connection can be provided between the outlet (5) and a scope to be cleansed, wherein the outlet (5) is designed to receive one of a series of mutually different adapters (6), each adapter being of a particular type that is characteristic for a particular type of scope, and the outlet (5) being arranged with detection means (7) to detect the type of adapter (6) that is connected to the outlet (5). The invention also relates to an adapter for connecting a scope to said apparatus, which adapter (6) comprises one or more features that are characteristic for the adapter and detectable with detection means of the apparatus (1).

## Description

The invention relates to a combination of an apparatus for cleansing a scope, such as an endoscope, and an adapter for connecting the scope to the apparatus, said apparatus being equipped with a source for supplying cleansing fluid, and said apparatus comprising a single outlet to which said source for cleansing fluid is connected, wherein through the adapter a fluid connection can be provided between the outlet and the scope to be cleansed.

The website https://comprex-medical.com/ teaches that reprocessing of flexible endoscopes is a major challenge in the daily routine of practices and clinics. To ensure correct cleaning, a complex workflow is required. Manual pre-cleaning of endoscopes with brushes takes a huge effort concerning labour and time. To avoid this manual precleaning it is taught to apply impulse flushing in one system, i.e. one apparatus to easily clean all the channels of flexible endoscopes in one shot.

In order to use the known cleansing apparatus, the scope to be cleansed must be identified and connected to the apparatus in a leakproof fashion. Then a fully automated cleansing process can be started which takes about five minutes, after which the scope can be removed from the apparatus for further handling and use. In particular the first step to follow is a high-level disinfection.

A problem that the invention seeks to solve is that there exist many different types of scopes they may be cleansed, each type requiring its own processing in terms of precleaning.

It is therefore an object of the invention to improve the reliability of the known way of precleaning, and to provide features which guarantee an automatic recognition of the scope that is connected to the apparatus for cleansing.

The invention is embodied in the features of one or more of the appended claims.

According to a first aspect of the invention, the single outlet of the apparatus for cleaning a scope is designed to receive the adapter with which the scope is connected to the apparatus, said adapter being selected as one of a series of mutually different adapters, and said adapter being of a particular type that is characteristic for a particular type of scope that the adapter connects to the apparatus, wherein the outlet is arranged with detection means to detect the type of adapter that is connected to the outlet. Accordingly it is possible to reliably detect which type of scope is connected to the cleansing apparatus, which enables to process the concerning scope according to its requirements.

Suitably the detection means comprises one or more sensors for detecting one or more characteristic features of an adapter that is connectable to the outlet. In a preferred embodiment the one or more sensors comprise a Hall-sensor to detect the presence or nonpresence of a magnet or magnets in the adapter.

The number of scopes and adapters to be detected can be increased by arranging that the one or more sensors comprise a Hall-sensor to detect not only the presence, but also the location of a magnet or magnets in the adapter.

In a practical embodiment the detection means comprise at least four Hall-sensors to detect the presence or nonpresence, and in case of presence the location of a magnet or magnets in the adapter.

In a further aspect of the invention the apparatus comprises a database providing the concurrence of the type of scope that is connectable to the apparatus for cleaning, and the adapter that corresponds with the concerning type of scope that is to be or is connected to the apparatus for cleaning. In this way the apparatus provides the information that is required to ensure the compatibility between the adapter and the connected scope, because the connection to each scope is different. It also supports the suitability of the cleaning program of the cleansing apparatus as will be applied to the connected type of scope.

The identification of the scope to be connected to the apparatus of the invention is supported by arranging that the apparatus has a scanning facility for scanning and detecting the type of scope to be connected to the apparatus for cleaning.

The security of the precleaning operation is promoted by the feature that the apparatus has signaling means for warning a user when no adapter or no suitable adapter is connected to the apparatus, and/or for indicating that an adapter is connected to the apparatus that corresponds to a selected type of scope to be cleansed.

It is further desirable that the apparatus has a security feature to detect whether the adapter that is connected to the outlet is properly connected so as to avoid leakage and unsafe operating conditions.

Desirably for the above purpose the apparatus comprises a sensor which is dedicated to detect a magnet in a turning ring of the adapter which is connectable to the outlet so as to enable detection the proper placement of the adapter.

Within the scope of the invention the adapter comprises one or more features that are characteristic for the adapter and detectable with the detection means of the apparatus.

Suitably the one or more features of the adapter comprise one or more magnets at predefined locations within the adapter.

In a practical embodiment the adapter comprises at least four magnets at predefined locations.

The accompanying drawing, which is incorporated into and forms a part of the specification, illustrates one or more embodiments of the present invention and, together with the description, serves to explain the principles of the invention. The drawing is only for the purpose of illustrating one or more embodiments of the invention and is not to be construed as limiting the invention.

In the drawing:
- figure 1 depicts an apparatus forming part of the combination of the apparatus and an adapter according to the invention; and
- figure 2 depicts an adapter forming part of the combination of the apparatus and the adapter according to the invention; and
- Figure 3 shows the combination of the apparatus and the adapter according to the invention.

Whenever in the figures the same reference numerals are applied, these numerals refer to the same parts.

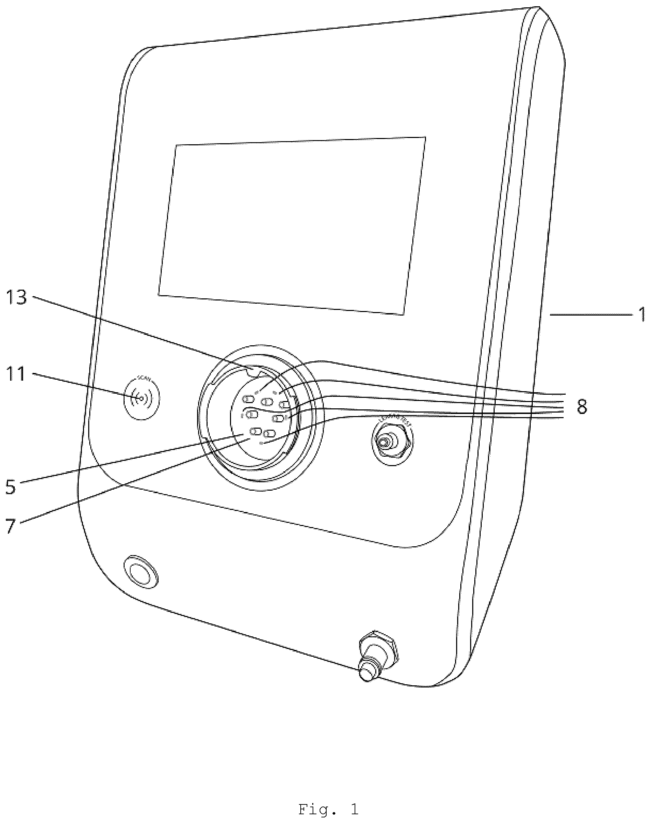

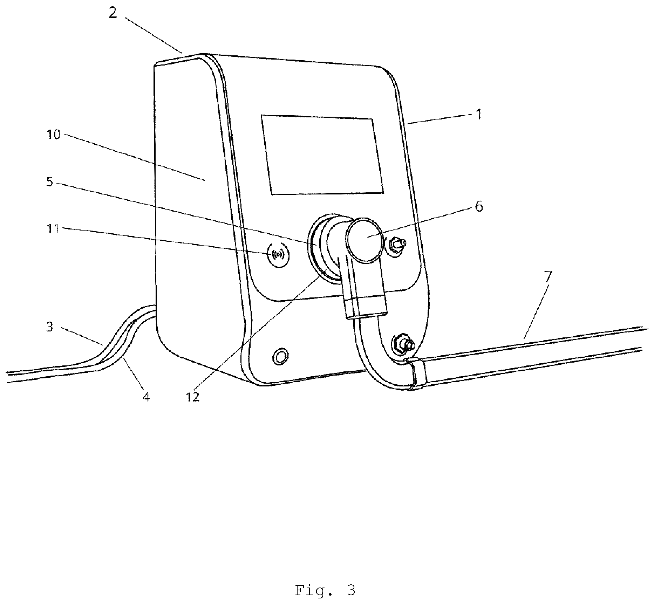

Figure 1 shows an apparatus 1 for cleansing scopes, such as endoscopes, the apparatus 1 comprises a housing 2 being equipped with a source 3 for supplying cleansing fluid as shown in figure 3. Figure 3 also shows an air supply line 4. The fluid supply line 3 and the air supply line 4 are used in combination to provide a pulsating mixture at the outlet 5 shown in figure **1****.** The manner in which the pulsating mixture are derived from the fluid and air that is supplied through the lines 3, 4 forms part of the prior art and is therefore not depicted in the figures.

Figure 3 depicts that the pulsating mixture which is derived from the fluid and air that is supplied through the lines 3, 4 comes available at the outlet 5 and that a fluid connection can be provided between the outlet 5 and a (not shown) scope to be cleansed. The scope comes in many varieties but as such is part of the prior art and is therefore not shown in the figures. The scope (not shown, but on the right-hand side of the apparatus 1 shown in figure 3) is connected through a line 7 with an adapter 6 to the outlet 5 of the apparatus 1. Said outlet 5 is designed to receive one of a series of mutually different adapters, each adapter 6 being of a particular type that is characteristic for a particular type of scope that may be cleansed. In connection therewith the outlet 5 is arranged with detection means 7 to detect the type of adapter 6 that is connected to the outlet 5.

Suitably the detection means 7 comprises one or more sensors 8 for detecting one or more characteristic features of the adapter 6 that is connected to the outlet 5. In turn the adapter 6 comprises one or more magnets 9 to be detected with the sensors 8 of the outlet 5. Typically the one or more sensors 8 comprise a Hall-sensor to detect the presence or nonpresence of a magnet or magnets 9 in the adapter 6.

Preferably the one or more sensors comprise a Hall-sensor to detect not only the presence or no presence, but also the location of a magnet or magnets 9 in the adapter 6.

Desirably the detection means comprise at least four Hall-sensors to detect the presence or nonpresence, and in case of presence the location of a magnet or magnets 9 in the adapter 1. This makes it possible to distinguish 32 different types of adapters, and correspondingly 32 different types of scopes that may be cleansed with the apparatus 1 of the invention.

Suitably the apparatus 1 comprises a database within its housing 10 providing the concurrence of the type of scope that is connectable to the apparatus 1 for cleaning, and the adapter 6 that corresponds with the concerning type of scope that is to be or is connected to the apparatus 1 for cleansing.

Preferably the apparatus 1 has a scanning facility 11 for scanning and detecting the type of scope to be connected to the apparatus 1 for cleaning.

Suitably the apparatus 1 has signaling means 12 for warning a user when no adapter or no suitable adapter is connected to the apparatus 1, and/or for indicating that an adapter 6 is connected to the apparatus 1 that corresponds to a selected type of scope to be cleansed.

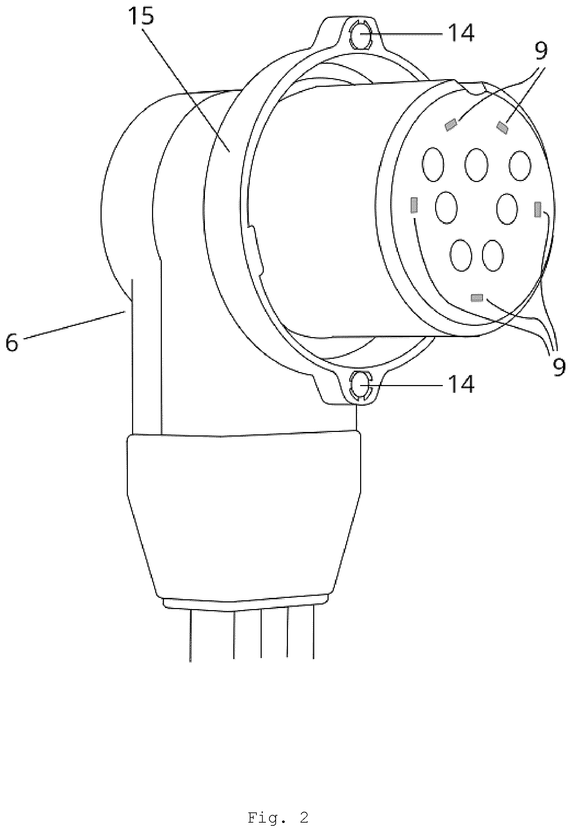

Furthermore it is preferable that the apparatus 1 has a security feature to detect whether the adapter 6 that is connected to the outlet 5 is properly connected, so as to avoid leakage and safe operating conditions. A preferable embodiment is shown in figure 1 and figure 2, which figures depict that the apparatus 1 comprises a sensor 13 which is dedicated to detect a magnet 14 in a turning ring 15 of the adapter 1 which is connectable to the outlet 5 so as to enable detection the proper placement of the adapter 6.

From the above description it will be clear that corresponding to the features of the apparatus 1, the adapter 6 comprises one or more features that are characteristic for the adapter 6 and detectable with detection means of the apparatus.

As mentioned above the one or more features comprise one or more magnets 9 at predefined locations within the adapter 6. the adapter comprises at least four magnets 9 at predefined locations within the adapter 6.

Although the invention has been discussed in the foregoing with reference to an exemplary embodiment of the invention, the invention is not restricted to this particular embodiment which can be varied in many ways without departing from the invention. The discussed exemplary embodiment shall therefore not be used to construe the appended claims strictly in accordance therewith. On the contrary the embodiment is merely intended to explain the wording of the appended claims without intent to limit the claim to this exemplary embodiment. The scope of protection of the invention shall therefore be construed in accordance with the appended claims only, wherein a possible ambiguity in the wording of the claims shall be resolved using this exemplary embodiment.

Variations and modifications of the present invention will be obvious to those skilled in the art and it is intended to cover in the appended claims all such modifications and equivalents. The entire disclosures of all references, applications, patents, and publications cited above are hereby incorporated by reference. Unless specifically stated as being "essential" above, none of the various components or the interrelationship thereof are essential to the operation of the invention. Rather, desirable results can be achieved by substituting various components and/or reconfiguration of their relationships with one another.

## Claims

1. A combination of an apparatus (1) for cleansing a scope, such as an endoscope, and an adapter for connecting the scope to the apparatus, said apparatus (1) being equipped with a source (3) for supplying cleansing fluid, and said apparatus (1) comprising a single outlet (5) to which said source (3) for cleansing fluid is connected, wherein through the adapter (6) a fluid connection can be provided between the outlet (5) and the scope to be cleansed, **characterized in that** the single outlet (5) is designed to receive the adapter (6), said adapter (6) being selected as one of a series of mutually different adapters (6), and said adapter being of a particular type that is characteristic for a particular type of scope that the adapter (6) connects to the apparatus (1), wherein the outlet (5) is arranged with detection means (7) to detect the type of adapter (6) that is connected to the outlet (5).

2. The combination of claim 1, **characterized in that** the detection means (7) comprises one or more sensors (8) for detecting one or more characteristic features of the adapter (6) that is connectable to the outlet (5).

3. The combination of claim 2, **characterized in that** the one or more sensors (8) comprise a Hall-sensor to detect the presence or nonpresence of a magnet or magnets (9) in the adapter (6).

4. The combination of claim 2 or claim 3, **characterized in that** the one or more sensors (8) comprise a Hall-sensor to detect the location of a magnet or magnets (9) in the adapter (6).

5. The combination according to any one of claims 1 - 4, **characterized in that** the detection means (7) comprise at least four Hall-sensors to detect the presence or nonpresence, and in case of presence the location of a magnet or magnets (9) in the adapter (6).

6. The combination according to any one of claims 1 - 5, **characterized in that** the apparatus (1) comprises a memory holding a database providing the concurrence of the type of scope that is connectable or connected to the apparatus (1) for cleansing, and the adapter (6) that corresponds with the concerning type of scope that is or may be connected to the apparatus (1) for cleansing.

7. The combination according to any one of claims 1 - 6, **characterized in that** the apparatus (1) has a scanning facility for scanning and detecting the type of scope to be connected to the apparatus (1) for cleansing.

8. The combination according to any one of claims 1 - 7, **characterized in that** the apparatus (1) has signaling means (12) for warning a user when no adapter or no suitable adapter is connected to the apparatus (1), and/or for indicating that an adapter (6) is connected to the apparatus (1) that corresponds to a selected type of scope to be cleansed.

9. The combination according to any one of claims 1 - 8, **characterized in that** the apparatus (1) has a security feature to detect whether the adapter (6) that is connected to the outlet (5) is properly connected.

10. The combination of according to claim 9, **characterized in that** the apparatus comprises a sensor (13) which is dedicated to detect a magnet (14) in a turning ring (15) of the adapter (6) which is connectable to the outlet (5) so as to enable detection the proper placement of the adapter (6).

11. An combination according to any one of claims 1 - 10, **characterized in that** the adapter (6) comprises one or more features that are characteristic for the adapter and detectable with detection means of the apparatus (1).

12. The combination according to claim 11, **characterized in that** the one or more features comprise one or more magnets (9) at predefined locations within the adapter (1).

13. The combination according to claim 11 or claim 12, **characterized in that** the adapter (6) comprises at least four magnets (9) at predefined locations.
